# EUROPÄISCHE PATENTANMELDUNG

(11) **EP 1 023 874 A2**
(43) Veröffentlichungstag der Anmeldung: **02.08.2000**
(21) Anmeldenummer: 99125121.6
(22) Anmeldetag: 16.12.1999
(51) Int. Cl.: A61B 18/20

(54) **Halterung für die Laseroptik eines Lasers zur Behandlung der Haut**

(30) Priorität: 28.01.1999 DE 19903318
(71) Anmelder: expo-med jezischek KG, 84549 Engelsberg (DE)
(72) Erfinder: Jezischek, Heinz, 84549 Engelsberg (DE)
(74) Vertreter: Laufhütte, Dieter, Dr.-Ing.

(57) **Zusammenfassung**

Die vorliegende Erfindung betrifft eine Halterung für die Laseroptik eines Lasers, insbesondere für eine Laseroptik eines Lasers zur Behandlung der Haut eines Patienten. Ein konstanter Abstand zwischen der Laseroptik und dem zu behandelnden Hautbereich wird erfindungsgemäß dadurch erhalten, daß die Halterung die Laseroptik sowie eine Erfassungsvorrichtung zur Erfassung des von dem behandelten Hautbereich ausgehenden Lichtes aufweist und die Halterung als geschlossener, einen Hohlraum begrenzender Körper ausgeführt ist, der eine oder mehrere Öffnungen zur Aufnahme der Laseroptik sowie der Erfassungseinrichtung aufweist und der eine weitere Öffnung umfaßt, durch die der die Laseroptik verlassende Laserstrahl geführt wird und deren diese begrenzender Randbereich auf den zu behandelnden Hautbereich aufsetzbar ist.

## Beschreibung

Die vorliegende Erfindung betrifft eine Halterung für die Laseroptik eines Lasers, insbesondere für die Laseroptik eines Lasers zur Behandlung der Haut eines Patienten.

Bei der Behandlung der Haut eines Patienten mittels Laserstrahlen kommt dem Abstand der Laseroptik vom dem zu behandelnden Hautbereich eine wesentliche Bedeutung zu. Der Abstand ist für den zu erzielenden Behandlungserfolg von erheblicher Bedeutung und muß daher möglichst exakt eingehalten werden. Vorbekannte Vorrichtungen weisen im allgemeinen ein Distanzstück auf, das an der Laseroptik bzw. an einer diese aufnehmenden Halterung befestigt ist und mittels dessen der gewünschte Abstand gewährleistet wird. Ist es erforderlich, den Abstand zu verändern, wird das verwendete Distanzstück durch ein entsprechend kürzer oder länger ausgeführtes Distanzstück ausgewechselt. Durch die auf diese Weise realisierbaren unterschiedlichen Abstände von Laseroptik und Haut wird die Wirkungsweise und die Eindringtiefe des Laserstrahls kontrolliert, wodurch unterschiedliche Behandlungsarten ermöglicht werden.

Vorbekannte Distanzstücke sind im allgemeinen aus Metall in Form eines Stiftes ausgeführt. Alternativ dazu werden Ausführungsformen aus Kunststoff verwendet. Um eine gute Einsehbarkeit der Behandlungsstelle zu gewährleisten, sind die vorbekannten Distanzstücke sehr dünn gehalten. Dadurch ergibt sich einerseits der Vorteil einer guten Zugänglichkeit und Einsehbarkeit des behandelten Hautbereiches, andererseits ergibt sich der Nachteil, daß die Distanz zwischen der Laseroptik und dem Auftreffpunkt des Lasers sehr stark davon abhängt, in welchem Winkel die das Distanzstück auf die Haut aufgesetzt wird. Darüber hinaus hängt der Abstand auch davon ab, in welcher Position sich das Distanzstück relativ zu der Laseroptik befindet. Ist das Distanzstück oberhalb der Laservorrichtung angeordnet ergibt sich bei gleichem Aufsetzwinkel auf der Haut ein anderer Abstand, als beispielsweise bei seitlicher Anordnung des Distanzstückes relativ zur Laseroptik.

Daraus ergibt sich der Nachteil, daß mittels vorbekannter Distanzstücke lediglich die Einhaltung eines ungefähren Abstandes realisierbar ist, während die exakte Einstellung und auch Einhaltung des Abstandes während der Behandlung nicht oder nur schwer möglich ist.

Es ist daher die Aufgabe der vorliegenden Erfindung eine Halterung für die Laseroptik eines Lasers zu schaffen, durch die der Abstand zwischen Laseroptik und dem Auftreffpunkt des Laserstrahls auf der Haut zuverlässig einstellbar ist und während der Behandlung beibehalten werden kann.

Diese Aufgabe wird dadurch gelöst, daß die Halterung die Laseroptik sowie eine Erfassungseinrichtung zur Erfassung des von dem behandelten Hautbereich ausgehenden Lichtes aufweist und daß die Halterung als geschlossener, einen Hohlraum begrenzenden Körper ausgeführt ist, der eine oder mehrere Öffnungen zur Aufnahme der Laseroptik sowie der Erfassungseinrichtung aufweist und der eine weitere Öffnung umfaßt, durch die der die Laseroptik verlassende Laserstrahl geführt wird und deren diese begrenzender Randbereich auf den zu behandelnden Hautbereich aufsetzbar ist.

Die als Handgriff dienende Halterung wird mittels des den die weitere Öffnung umgebenden Randbereichs auf die Haut des Patienten aufgesetzt. Die Verwendung eines zusätzlich vorzusehenden Distanzstückes entfällt. Der gewünschte Abstand zwischen Laseroptik und Haut wird erfindungsgemäß zuverlässig und auf einfache Weise dadurch realisiert, daß die Laseroptik in einem definierten Abstand zu der auf die Haut aufzusetzenden Öffnung der Halterung angeordnet ist.

Die oben beschriebenen Nachteile, die im wesentlichen darauf zurückzuführen sind, daß der Abstand einerseits vom Aufsetzwinkel des Distanzstückes und andererseits von der Positionierung des Distanzstückes relativ zur Laseroptik abhängt, werden durch die erfindungsgemäße Ausgestaltung der Halterung zuverlässig vermieden.

In weiterer Ausgestaltung der vorliegenden Erfindung ist vorgesehen, daß die Halterung als Hohlzylinder ausgeführt ist und sich die Öffnungen zur Aufnahme der Laseroptik sowie der Erfassungseinrichtung in einer Stirnseite und die weitere Öffnung in der anderen Stirnseite des Hohlzylinders befinden. Der Laserstrahl durchläuft den Hohlzylinder, tritt durch die weitere Öffnung der Halterung hindurch und trifft schließlich auf dem zu behandelnden Hautbereich auf.

Besonders vorteilhaft ist es, wenn der die weitere Öffnung begrenzende Randbereich der Halterung in einer Ebene liegt, deren Senkrechte nicht parallel, sondern in einem Winkel zur Längsachse der Halterung angeordnet ist. Die Halterung kann entsprechend auf der auf der Haut aufzusetzenden Seite in einem beliebigen Winkel angeschrägt werden.

Der Abstand der Laseroptik von der weiteren Öffnung kann variierbar sein, wobei eine Meßeinrichtung vorgesehen ist, mittels derer der Abstand bestimmbar ist. Auf diese Weise kann der gewünschte Abstand exakt eingestellt und während der Behandlung angezeigt werden. Eine Feineinstellung des Abstandes von außen ist problemlos möglich.

Die Meßeinrichtung kann als Skala ausgeführt sein und auf der Laseroptik angeordnet sein.

In weiterer Ausgestaltung der vorliegenden Erfindung ist vorgesehen, daß die den Hohlraum der Halterung begrenzenden Wandungen aus einem lichtundurchlässigen Material bestehen. Hierdurch wird der Vorteil erreicht, daß der Laserstrahl von außen nicht sichtbar ist, wodurch das Tragen spezieller Schutzbrillen des behandelnden Personals überflüssig wird. Das Material kann Kunststoff, Metall oder auch jedes andere beliebige lichtundurchlässige Material umfassen. Bei entsprechendem Aufsetzen der Halterung auf die Haut wird der zu behandelnde Hautbereich lichtdicht abgegrenzt.

Gemäß einer bevorzugten Ausgestaltung der vorliegenden Erfindung ist vorgesehen, daß die Erfassungseinrichtung einen Lichtleiter umfaßt. Auf diese Weise wird der Vorteil erreicht, daß aufgrund der Verwendung eines leichten optischen Faserkabels die gesamte Halterung verhältnismäßig leicht ausgeführt ist und somit gut handhabbar ist. Der Nachteil der schwerfälligen Bedienbarkeit vorbekannter Lösungen mit Kamerakopf und Objektiv, die an einem Griff befestigt sind, wird somit vermieden.

Besonders vorteilhaft ist es, wenn der Lichtleiter derart ausgeführt ist, daß der zu behandelnde Hautbereich beleuchtet und das von diesem Bereich ausgehende Licht erfaßt wird. Somit ist eine separat vorzusehende Beleuchtung des Behandlungsbereiches, die bei Verwendung einer Mikrokamera erforderlich ist, nicht nötig. Das von dem Lichtleiter erfaßte Signal wird auf einen Monitor übertragen, der frei wählbar an einer vom Operateur gut einsehbaren Position aufgestellt werden kann. Durch die Größe des Monitors kann die gewünschte Bildgröße ausgewählt werden.

Gemäß einer bevorzugten Ausgestaltung der folgenden Erfindung ist ein Zoom-Objektiv vorgesehen, mittels dessen der zu behandelnde Hautbereich vergrößert darstellbar ist. Bei Verwendung eines Lichtleiters kann das Zoomobjektiv extern eingesetzt werden, so daß gewährleistet ist, daß der Operateur schnell und einfach den gewünschten Hautabschnitt vergrößern oder verkleinern kann.

Die Halterung kann eine Absaugvorrichtung umfassen, die zum Absaugen der in dem Hohlraum befindlichen Luft bzw. von darin gebildeten Gasen dient. Die abzusaugenden Gase entstehen während der Behandlung durch die sehr starke Erhitzung der Haut bzw. der Haare. Werden diese nicht abgesaugt führt dies zu die Behandlung beeinträchtigenden Sichtbehinderungen. Die Absaugeinrichtung kann in einer Öffnung der die Laseroptik sowie die Erfassungsvorrichtung aufnehmenden Stirnseite des Hohlzylinders angeordnet sein. Selbstverständlich ist es ebenso möglich, diese seitlich anzuordnen.

Die Absaugeinrichtung kann in einer geringen Größe eingesetzt werden, da der zwischen der Laseroptik und Haut befindliche Raum durch den Hohlkörper der erfindungsgemäßen Halterung begrenzt wird und somit die abzusaugende Luftmenge verhältnismäßig gering ist. Die Reinigung der abgesaugten Abgase kann beispielsweise mittels eines Aktivkohlefilters erfolgen.

Die Verwendung aus dem Stand der Technik bekannter großer Absauggeräte, die auch aus dem Umfeld des Behandlungsbereiches Luft absaugen und entfernen müssen und entsprechend ein leistungsfähiges und belästigende Geräusche erzeugendes Gebläse aufweisen, kann erfindungsgemäß entfallen. Hinzu kommt, daß vorbekannte Absauggeräte bedingt durch ihre Größe nicht überall einsetzbar sind, da sie oftmals das Blickfeld stören und somit eine optimale Behandlung erschweren. Insbesondere kann durch die erfindungsgemäße Halterung die Behandlung dahingehend vereinfacht werden, daß kein zusätzliches Personal erforderlich ist, um die entstehenden Gase abzusaugen.

Weitere Einzelheiten und Vorteile der Erfindung werden anhand eines in der Zeichnung dargestellten Ausführungsbeispiels näher erläutert. Es zeigen:
- Fig. 1:: eine schematische Längsschnittdarstellung der erfindungsgemäßen Halterung,
- Fig. 2:: eine schematische Querschnittsdarstellung der erfindungsgemäßen Halterung gemäß Fig. 1 und
- Fig. 3:: eine Halterung gemäß dem Stand der Technik.

Die Halterung 10 ist in der Gestalt eines Hohlzylinders ausgebildet und dient als vom Operateur während der Behandlung geführter Handgriff. Die Wandungen des Hohlzylinders begrenzen den Hohlraum 11. In der oben befindlichen Stirnseite des Hohlzylinders befinden sich die Öffnungen 12, 14, in denen die Laseroptik 20 sowie die Erfassungseinrichtung 30 aufgenommen sind. Ebenfalls in der oben angeordneten Stirnseite befindet sich die Öffnung 16, in der die Absaugeinrichtung 50 angeordnet ist.

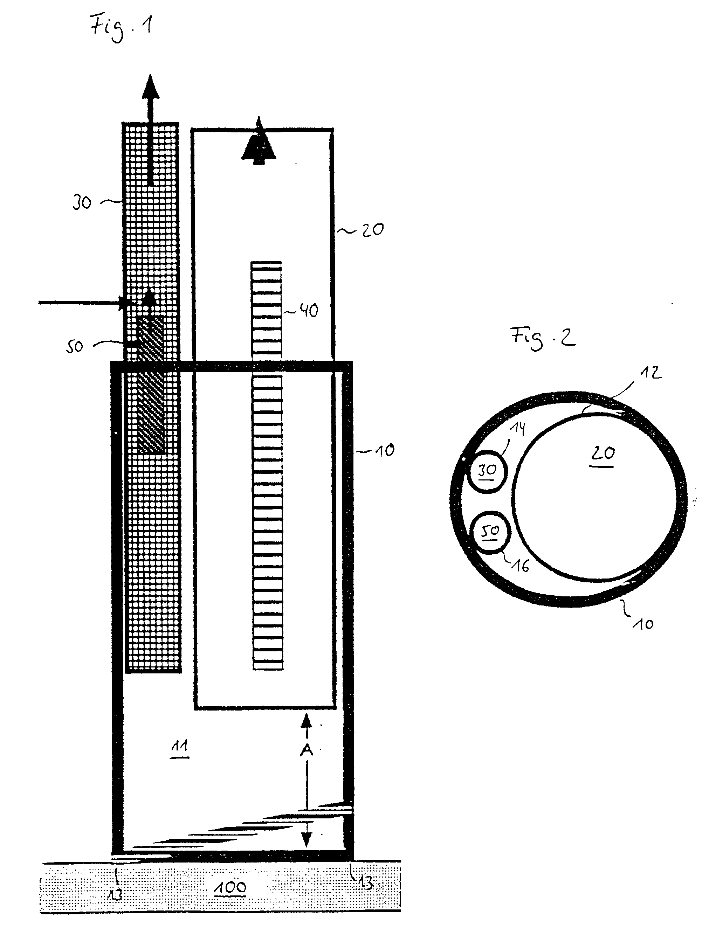

Die Laseroptik 20 weist die Meßeinrichtung 40 auf, die als Skala ausgeführt ist. Diese ermöglicht die Überprüfung und Einstellung des gewünschten Abstandes A zwischen der Laseroptik 20 sowie der in der unten angeordneten Stirnseite des Hohlzylinders befindlichen weiteren Öffnung. In der aus Fig. 1 ersichtlichen Position liegt die unten angeordnete Stirnseite des Hohlzylinders, die den Randbereich 13 der weiteren Öffnung bildet, vollständig und lichtdicht auf der Haut 100 des Patienten auf. Die Mantelfläche des Hohlzylinders besteht aus einem lichtundurchlässigen Material.

Die untere Stirnseite des Hohlzylinders kann auch schräg ausgeführt werden, wie dies durch die gestrichelte Linie in Fig. 1 angedeutet ist.

Zum Betrieb der erfindungsgemäßen Halterung wird der Hohlkörper 10 auf die Haut 100 des Patienten aufgesetzt und mittels der Skalierung 40 der gewünschte Abstand eingestellt. Der im oberen Endbereich der Laseroptik 20 dargestellte Pfeil kennzeichnet die Verbindung der Laseroptik 20 zum nicht dargestellten Laser. Der Laserstrahl kann mittels eines Armes und Spiegels oder auch mittels eines optischen Faserkabels übertragen werden.

Während der Behandlung wird mittels der als Lichtleiter ausgeführten Erfassungseinrichtung 30 die zu behandelnde Stelle 100 der Haut beleuchtet. Gleichzeitig erfolgt ebenfalls mittels des Lichtleiters die optische Erfassung der Behandlungsstelle 100. Die aufgenommenen Signale werden mittels des Lichtleiters an einen geeignet anzuordnenden Monitor weitergeleitet, wie dies durch den im oberen Endbereich des Lichtleiters angeordneten Pfeil angedeutet ist. Vorteilhaft ist ein Zoom-Objektiv vorgesehen, mittels dessen der Bediener den zu untersuchenden Bereich vergrößern oder verkleinern kann. Auf diese Weise entfällt die bei vorbekannten Lösungen gegebene Notwendigkeit, die Vergrößerung mittels Lupenleuchten, Lupenbrillen oder mittels in unterschiedlichen Blickwinkeln angeordneten Kameras zu erzielen.

Die Absaugeinrichtung 50 der erfindungsgemäßen Halterung 10 saugt die in dem Hohlraum 11 vorhandenen Gase während der Behandlung ab. Wie dies ist durch die im Bereich der Absaugeinrichtung 50 dargestellten Pfeile in Fig. 1 angedeutet ist, ist die Absaugeinrichtung 50 mit einer Absaugpumpe verbunden. Aufgrund der Tatsache, daß die Absaugeinrichtung 50 nicht wie bei vorbekannten Lösungen den Umgebungsbereich der Behandlungsstelle 100, sondern nur den Hohlraum 11 des Hohlzylinders 10 absaugt, kann diese verhältnismäßig klein ausgeführt werden.

Fig. 2 zeigt eine schematische Querschnittsdarstellung der erfindungsgemäßen Halterung gemäß Fig. 1 und verdeutlicht die Anordnung der Öffnungen 12, 14 und 16 sowie der Laseroptik 20, der Erfassungseinrichtung 30 sowie der Absaugeinrichtung 50.

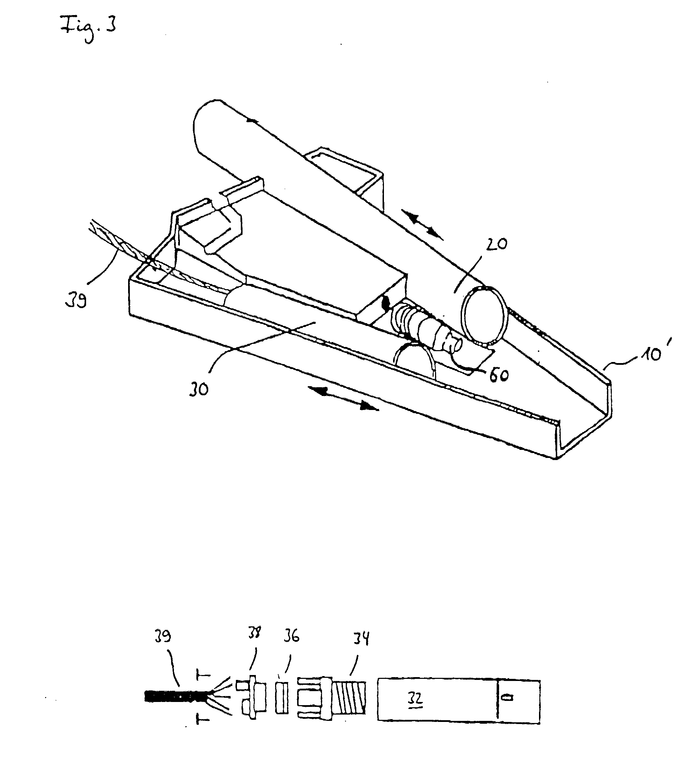

Fig. 3 zeigt in einer perspektivischen Ansicht eine schematische Darstellung einer Halterung 10' gemäß dem Stand der Technik.

Hier dargestellt ist die Laseroptik 20 sowie eine Erfassungseinrichtung 30, die als Videokamera ausgeführt ist. Der verhältnismäßig komplizierte Aufbau der Videokamera ergibt sich aus Figur 3, unten. Die dort dargestellte Videokamera besteht aus dem Objektiv 32, der CCD-Einheit 38, der zugehörigen Halterung 34, dem Infrarotfilter 36 sowie dem Anschlußkabel 39.

Durch den erfindungsgemäß vorgesehenen Einsatz eines Lichtleiters erübrigt sich zum einen die Verwendung einer derartigen Videokamera und darüber hinaus der Einsatz des entsprechend schweren, im allgemeinen als 18-poliges Übertragungskabel ausgeführten Kabels 39. Statt dessen können erfindungsgemäß Glasfaserkabel eingesetzt werden, die leicht und dünn sind und mit Schutzhülle einen Außendurchmesser von ca. 3 mm aufweisen.

Als weiterer Nachteil einer Ausführung gemäß dem Stand der Technik ergibt sich, daß die als Videokamera ausgeführte Erfassungseinrichtung 30 exakt im richtigen Winkel zum Laserstrahl eingestellt werden muß.

Zwischen der Laseroptik 20 sowie der Erfassungseinrichtung 30 befindet sich, wie aus Fig. 3 ersichtlich, die Beleuchtungseinheit 60, die zur Beleuchtung des Sichtfeldes dient. Eine derartige Beleuchtungseinheit ist bei der erfindungsgemäß möglichen Verwendung eines Lichtleiters überflüssig.

## Patentansprüche

1. Halterung (10) für die Laseroptik (20) eines Lasers, insbesondere für die Laseroptik (20) eines Lasers zur Behandlung der Haut (100) eines Patienten,
wobei die Halterung die Laseroptik (20) sowie eine Erfassungseinrichtung (30) zur Erfassung des von dem behandelten Hautbereich (100) ausgehenden Lichtes aufweist,
und wobei die Halterung (10) als geschlossener, einen Hohlraum (11) begrenzenden Körper ausgeführt ist, der eine oder mehrere Öffnungen (12, 14) zur Aufnahme der Laseroptik (20) sowie der Erfassungseinrichtung (30) aufweist und der eine weitere Öffnung umfaßt, durch die der die Laseroptik (20) verlassende Laserstrahl geführt wird und deren diese begrenzender Randbereich (13) auf den zu behandelnden Hautbereich (100) aufsetzbar ist.

2. Halterung (10) nach Anspruch 1, dadurch gekennzeichnet, daß die Halterung (10) als Hohlzylinder ausgeführt ist und sich die Öffnungen (12, 14) zur Aufnahme der Laseroptik (20) sowie der Erfassungseinrichtung (30) in einer Stirnseite und die weitere Öffnung in der anderen Stirnseite des Hohlzylinders befinden.

3. Halterung (10) nach Anspruch 1 oder 2, dadurch gekennzeichnet, daß der die weitere Öffnung begrenzende Randbereich (13) der Halterung (10) in einer Ebene liegt, deren Senkrechte nicht parallel, sondern in einem Winkel zur Längsachse der Halterung (10) angeordnet ist.

4. Halterung (10) nach einem oder mehreren der Ansprüche 1 bis 3, dadurch gekennzeichnet, daß der Abstand (A) der Laseroptik (20) von der weiteren Öffnung variierbar ist und eine Meßeinrichtung (40) vorgesehen ist, mittels derer der Abstand (A) bestimmbar ist.

5. Halterung (10) nach Anspruch 4, dadurch gekennzeichnet, daß die Meßeinrichtung als Skala ausgeführt ist und auf der Laseroptik (20) angeordnet ist.

6. Halterung (10) nach einem oder mehreren der Ansprüche 1 bis 5, dadurch gekennzeichnet, daß die den Hohlraum (11) der Halterung (10) begrenzenden Wandungen aus einem lichtundurchlässigen Material bestehen.

7. Halterung (10) nach einem oder mehreren der Ansprüche 1 bis 6, dadurch gekennzeichnet, daß die Erfassungseinrichtung (30) einen Lichtleiter umfaßt.

8. Halterung (10) nach Anspruch 7, dadurch gekennzeichnet, daß der Lichtleiter derart ausgeführt ist, daß der zu behandelnde Hautbereich (100) beleuchtet und das von diesem Bereich (100) ausgehende Licht erfaßt wird.

9. Halterung (10) nach einem oder mehreren der Ansprüche 1 bis 8, dadurch gekennzeichnet, daß ein Zoom-Objektiv vorgesehen ist, mittels dessen der behandelte Hautbereich (100) vergrößert darstellbar ist.

10. Halterung (10) nach einem oder mehreren der Ansprüche 1 bis 9, dadurch gekennzeichnet, daß eine Absaugeinrichtung (50) zum Absaugen der in dem Hohlraum (11) befindlichen Luft vorgesehen ist.

11. Halterung (10) nach Anspruch 2 und 10, dadurch gekennzeichnet, daß die Absaugeinrichtung (50) in einer Öffnung (16) der die Laseroptik (20) sowie die Erfassungseinrichtung (30) aufnehmenden Stirnseite des Hohlzylinders angeordnet ist.
